# EUROPEAN PATENT APPLICATION

(11) **EP 2 267 436 A1**
(43) Date of publication of application: **29.12.2010**
(21) Application number: 09725661.4
(22) Date of filing: 25.03.2009
(51) Int. Cl.: G01N 21/76, G01N 33/553

(54) **SUBSTRATE FOR USE IN IMMOBILIZING SUBSTANCE, SUBSTRATE WITH SUBSTANCE IMMOBILIZED THEREON, AND ASSAY METHOD**

(30) Priority: 26.03.2008 JP 2008080292
(71) Applicant: Riken, Wako-shi, Saitama 351-0198 (JP)
(72) Inventor: ITO, Yoshihiro, Wako-shi Saitama 351-0198 (JP); TASHIRO, Hideo, Wako-shi Saitama 351-0198 (JP)
(74) Representative: Polypatent
(86) International application number: PCT/JP2009/001317
(87) International publication number: WO 2009/119082

(57) **Abstract**

The present invention relates to a substance-immobilizing substrate for immobilizing a substance to be detected by chemiluminescence, The substance-immobilizing substrate of the present invention comprises a metal portion composed of at least one metal selected from the group consisting of chromium and molybdenum, or an alloy of the metal on at least a portion of a surface of the substrate. The present invention further relates to a substance-immobilized substrate wherein a substance is immobilized on the substance-immobilizing substrate. The substance is immobilized on the metal portion and the substrate is used for detecting the immobilized substance by chemiluminescence. The present invention further relates to an analysis method comprising directly or indirectly detecting by chemiluminescence a substance that is immobilized on a substrate. The substrate comprises a portion composed of at least one metal selected from the group consisting of chromium and molybdenum, or an alloy of the metal on at least a portion of a surface of the substrate, and the substance is immobilized on the metal portion.

## Description

### CROSS-REFERENCE TO RELATED APPLICATIONS

This application claims the benefit of priority to Japanese Patent Application No. 2008-080292, filed on March 26, 2008, which is expressly incorporated herein by reference in its entirety.

### TECHNICAL FIELD

The present invention relates to a substance-immobilizing substrate for detecting, with high sensitivity and by chemiluminescence, a substance to be immobilized such as a polypeptide, nucleic acid, or lipid, and to a substance-immobilized substrate in which a substance such as a polypeptide, nucleic acid, or lipid is immobilized on the above substrate, permitting the detection with high sensitivity of the substance by chemiluminescence. The present invention further relates to an analysis method comprising a subustance by chemiluminescence.

### BACKGROUNDA RT

Immunoplates for immunological detection on which antibodies or antigens are immobilized, DNA chips comprising nucleic acids immobilized on chips, and the like have been widely employed in recent years, Methods for detecting the substance that is immobilized on the substrate include chemiluminescence, fluorescence, and coloration, Of these, chemiluminescence is a widely employed method because it permits detection with high sensitivity and can be performed with an inexpensive detection device.

Nitrocellulose-coated glass substrates are currently commercially available as substrates for detecting biomolecules by chemiluminescence, Gold-coated glass substrates and substrates of plastics such as polyethylene terephthalate, polycarbonate, and acrylic are known substrates used in chemiluminescence (for example, see Japanese Unexamined Patent Publication (KOKAI) Nos, 2007-228905 and 2007-195431, which are expressly incorporated herein by reference in their entirety).

However, the above substrates that have been conventional employed in chemiluminescence do not necessary afford adequate sensitivity, Thus, there is need for a means permitting analysis by chemiluminescence with higher sensitivity.

### DISCLOSURE OF THE INVENTION

Accordingly, the object of the present invention is to provide a means for detecting, with high sensitivity and by chemiluminescence, substances such as polypeptides, nucleic acids, and lipids.

The present inventors conducted extensive research into achieving the above object, resulting in the discovery that a substrate having a chromium and/or molybdenum layer on the surface thereof made it possible to achieve this object, and devised the present invention.

An aspect of the present invention relates to a substance-immobilizing substrate for immobilizing a substance to be directly or indirectly detected by chemiluminescence, comprising a metal portion composed of at least one metal selected from the group consisting of chromium and molybdenum, or an alloy of the metal on at least a portion of a surface of the substrate.

The substrate may comprise a layer of a polymer that is electrically neutral as a whole molecule on at least a portion of a surface of the metal portion.

The surface of the metal portion may be the surface that has been subjected to piranha processing and/or ozone treatment.

The substance to be immobilized may be at least one substance selected from the group consisting of polypeptides, nucleic acids, lipids, cells, and components thereof.

A further aspect of the present invention relates to a substance-immobilized substrate, wherein
a substance is immobilized on the above substance-immobilizing substrate,
the substance is immobilized on the metal portion, and
the substrate is used for directly or indirectly detecting the immobilized substance by chemiluminescence.

The substance may be at least one substance selected from the group consisting of polypeptides, nucleic acids, lipids, cells, and components thereof,

A still further aspect of the present invention relates to an analysis method comprising directly or indirectly detecting by chemiluminescence a substance that is immobilized on a substrate,
wherein the substrate comprises a metal portion composed of at least one metal selected from the group consisting of chromium and molybdenum, or an alloy of the metal on at least a portion of a surface of the substrate, and
the substance is immobilized on the metal portion,

The substrate may comprise a layer of a polymer that is electrically neutral as a whole molecule on at least a portion of a surface of the metal portion.

The surface of the metal portion may be subjected to piranha processing and/or ozone treatment prior to immobilizing the substrate on the metal portion.

The substance may be at least one substance selected from the group consisting of polypeptides, nucleic acids, lipids, cells, and components thereof.

In the analysis method, the substance that is immobilized on the substrate may be directly detected by chemiluminescence.

In the analysis method, the substance that is immobilized on the substrate may be indirectly detected by detecting a substance reacting specifically with the substance that is immobilized on the substrate by chemiluminescence.

The present invention permits the detection with high sensitivity of a substance immobilized on a substrate by chemiluminescence.

### BEST MODE FOR CARRYING:OUT THE INVENTION

The present invention relates to a substance-immobilizing substrate for immobilizing a substance to be directly or indirectly detected by chemiluminescence, The substance-immobilizing substrate of the present invention comprises a metal portion composed of at feast one metal selected from the group consisting of chromium and molybdenum, or an alloy of this metal, on at least a portion of the surface thereof. The fact that immobilization of the substance on chromium and molybdenum and detection by chemiluminescence of the substance that has been immobilized permits, detection with extremely high sensitivity was discovered by the present inventors through research. This was attributed to the fact that chromium and molybdenum have high reflectance.
The substance-immobilizing substrate of the present invention will be described in greater detail below.

In the substance-immobilzing substrate of the present invention, a metal portion composed of at least one metal selected from the group consisting of chromium and molybdenum, or an alloy of the metal, is present on at least a portion of the surface. In the substance-immobilizing substrate of the present invention, the entire substrate may be composed of the metal. From a practical perspective, it is desirable for a layer composed of the metal to be formed on a substrate composed of a known material.

The metal portion is formed on at least a portion of the surface of the substrate, and need only be formed in regions on which the substance is immobilized. Taking workability into account, it is desirably formed over the entire surface of the substrate. The metal can be molybdenum, chromium, an alloy of chromium and molybdenum, or an alloy of chromium and/or molybdenum with another metal. In the case of an alloy, it is desirable for the chromium and/or molybdenum to account for equal to or greater than 50 mass percent of the alloy The metal portion is preferably comprosed of chromium or molybdenum,

When the substance-immobilizing substrate of the present invention is the substrate having a metal layer on a base material, the base material is not specifically limited. For example, it can be composed of polystyrene, which is widely employed in microplates and the like; composed of an organic substance such as acrylic resin, polyvinyl chloride, polyethylene terephthalate, polycarbonate, or polypropylene; or a glass base. The form of the substrate is in no way limited. It can be platelike, such as a microarray substrate, or consist of holes or grooves provided in a plate, such as the wells of a microplate. The thickness of the substrate is, for example, 0.5 to 10 mm, desirably 1 to 5 mm. The thickness of the metal portion is, for example, 0.00001 to 0.01 mm, desirably 0.0001 to 0.0001 mm. When the entire substrate is composed of metal, the substrate can be molded by a known molding method. Additionally, in a substrate having a metal layer on a base material, the metal layer can be formed on the base material by a known film-forming method, such as vapor deposition. Another layer, such as an oxide layer, may be present between the metal layer and the base material The reflectance of the metal portion is desirably equal to or greater than 50 percent, preferably equal to or greater than 70 percent, and more preferably, 70 to 100 percent. The reflectance can be increased by a means such as subjecting the surface of the metal portion to mirror surface processing or increasing the smoothness of the substrate surface on which the metal layer is formed.

Before immobilizing the substance on the substrate surface, the surface of the metal portion can be preprocessed by subjecting it to an ozone treatment. The ozone treatment can be conducted by placing the substrate in an ozone atmosphere and then irradiating it with ultraviolet radiation. The ozone concentration in the atmosphere, the ultraviolet irradiation conditions, and the period of radiation can be suitably set. Preprocessing by piranha processing is also possible. "Piranha processing" refers to cleaning with a piranha solution consisting of a mixed solution of sulfuric acid and hydrogen peroxide solution. The piranha solution employed is desirably a mixed solution of sulfuric acid:30 mass percent hydrogen peroxide solution = 1:3 (mass ratio). Piranha processing can be conducted, for example, by immersing the entire substrate in piranha solution. Conducting such preprocessing further increases detection sensitivity.

In the substrate of the present invention, a layer of a polymer that is electrically neutral as a whole molecule is desirably present on at least a portion of the surface of the metal portion, preferably on the entire surface of the measurement region. The polymer layer can inhibit nonspecific adsorption and thus further increase detection sensitive. In the present invention, the phrase " electrically neutral as a whole molecule" means that there are no groups which are dissociated and become ions in an aqueous solution at close to neutral pH (for example, pH 6 to 8), or if there are, there are groups that become cations and groups that become anions, with the total electrical charge per molecule being essentially zero. In the present invention, the term "essentially" as relates to electrical charge means that the total charge is zero, or if not zero, a low degree that does not negatively impact the effect of the present invention.

The polymer is desirably water soluble. For a polymer, the term "water soluble" means, for example, that the solubility of the polymer in water (the number of grams dissolving per 100 g of water) is equal to or greater than five. When a polymer that is insoluble in water is employed, it becomes necessary to employ a non-aqueous solvent in addition to water or alcohol, and the non-aqueous solvent will sometimes denature the substance to be immobilized, Accordingly, in the present invention, to prevent denaturation of the substance to be immobilized, the use of a water-soluble polymer is desirable. A water-soluble polymer can afford the further advantage of inhibiting non-specific adsorption,

The water-soluble polymer that is electrically neutral as a molecule can be a bipolar or nonionic polymer. In this context, the term "bipolar" means having groups that become anions and groups that become cations when dissociated in an aqueous solution of near neutral pH (for example, pH 6 to 8), with the total Electrical charge per molecule of polymer being essentially zero. The term "nonionic" means essentially having no groups becoming ions when dissociated in an aqueous solution of near neutral pH (for example, pH 6 to 8). In this context, the phrase "essentially having no groups becoming ions when dissociated" means having no such groups, or if present, having them in such a small quantity (for example, the number of such groups being equal to or less than 1 percent of the number of carbon atoms) that they do not negatively impact the effect of the present invention. Of these, nonionic polymers afford the advantages of effective prevention of nonspecific adsorption, inexpensive manufacturing, and availability.

The number average molecular weight of the polymer is not specifically limited. Normally, it is about 350 to 5 million. When the molecular weight of the polymer is excessively high, considerable crosslinking occurs between polymer molecules. There are cases where the reaction between the substance to be immobilized and the substance (photocrosslinking agent or the like) provided to react with the substance to be immobilized tends not to occur. Thus, about 500 to hundreds of thousands is desirable.

The following are specific examples of nonionic polymers that can be employed in the present invention, but the present invention is not limited thereto, polyalkylene glycols such as polyethylene glycol (PEG) and polypropylene glycol; nonionic vinyl polymers having structural components in the form of one or a combination of monomer units such as vinyl alcohol, methyl vinyl ether, vinyl pyrrolidone, vinyl oxazolidone, vinyl methyl oxazolidone, 2-vinyl pyridine, 4-vinyl pyridine, N-vinyl succinimide, N-vinyl formamide, N-vinyl-N-methyl formamide, N-vinyl acetamide, N-vinyl-N-methyl acetamide, 2-hydroxyethyl methacrylate, polyethylene glycol methacrylate, polyethylene glycol acrylate, acrylamide, methacrylamide, N,N-dimethyl acrylamide, N-isopropyl acrylamide, diacetone acrylamide, methylolacrylamide, acryloyl morpholine, acryloyl pyrrolidine, acryloyl piperidine, styrene, chloromethyl styrene, bromomethyl styrene, vinyl acetate, methyl methacrylate, butyl acrylate, methyl cyanoacrylate, ethyl cyanoacrylate, n-propyl cyanoacrylate, isopropyl cyanoacrylate, n-butyl cyanoacrylate, isobutyl cyanoacrylate, tert-butyl cyanoacrylate, glycidyl methacrylate, ethyl vinyl ether, n-propyl vinyl ether, isopropyl vinyl ether, n-butyl vinyl ether, isobutyl vinyl ether, and tert-butyl vinyl ether; and natural polymers such as gelatin, casein, collagen, gum arabic, xanthan gum, traganth gum, guar gum, pullulan, pectin, sodium alginate, hyaluronic acid, chitosan, chitin derivatives, carrageenan, starches (carboxymethyl starch, aldehyde starch), dextrin, cyclodextrin, methyl cellulose, viscose, hydroxyethyl cellulose, hydroxyethyl methyl cellulose, carboxymethyl cellulose, hydroxypropyl cellulose, and other water-soluble derivatives. Further, commercially available products of photocrosslinking water-soluble polymers based on these polymers can also be employed, such as "AWP", made by Toyo Gosei Co., Ltd., that is based on polyvinyl alcohol. Of these, polyethylene glycol polymers are preferred, with vinyl polymers of polyethylene glycol (meth)acrylate being of greater preference. In the present invention, the term "(meth)acrylate" means acrylate or methacrylate. "(Meth)acrylic" means acrylic or methacrylic.

Phosphorylcholine-containing bipolar polymers, specifically the phosphorylcholine-containing bipolar polymer containing the unit having the structure denoted by general formula [I] below, can be employed as the polymer.

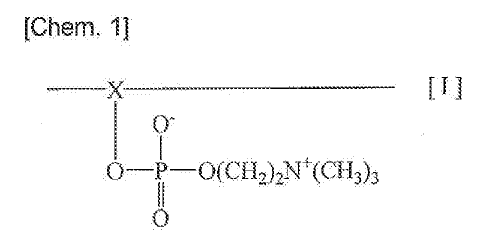

(In general formula [I], X denotes a polymerizable atom group in a polymerized state)

For the sake of simplicity, the unit having the structure denoted by general formula [1] above will be referred to as "unit [I]."

The phosphorylcholine contained in general formula [I] is a structural component of biomembranes. Biomembranes undergo little nonspecific adsorption despite coming into contact with a variety of substances. This has been attributed to the preventive effect of phosphorylcholine on nonspecific adsorption. In the present invention, the use of phosphorylcholine-containing polymer as the above polymer can effectively suppress nonspecific adsorption,

Unit [I] denotes a polymerizable atom group in a polymerized state in the form of a unit containing a phosphorylcholine group, X is desirably a vinyl monomer residue. Unit [I] is desirably the unit denoted by general formula [I'] below.

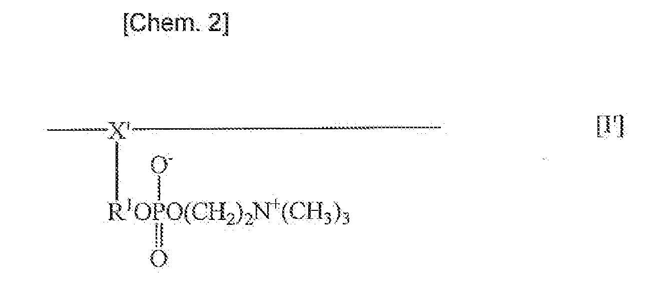

(Where X' denotes a methacryloxy group, methacrylamide group; acryloxy group, acrylamide group, and styrylamide group, with a vinyl moiety in a state of addition polymerization, and R¹ denotes a single bond or an alkylene group having 1 to 10 carbon atoms (optionally substituted with one or two hydroxyl groups)).

Desirable examples of such units are: units derived from (that is, to which such units are polymerized) 2-methacryloyloxyethyl phosphorylcholine, 2-acryloyloxyethyl phosphorylcholine, N-(2-methacrylamide)ethyl phosphorylcholine, 4-methacryloyloxybutyl phosphorylcholine, 6-methacryloyloxyhexyl phosphorylcholine, 10-methacryloyloxydecyl phosphorylcholine, ω-methacryloyldioxyethylene phosphorylcholine, and 4-styryloxybutyl phosphorylcholine. Of these, units derived from 2-methacryloyloxyethyl phosphorylcholine are preferred.

The polymer containing unit [I] may be comprised solely of unit [I], or may be a copolymer of unit [I] and other polymerizable monomers that do not negatively impact the effect of the present invention, A desirable example of another polymerizable monomer is a vinyl monomer, with (meth)acrylic acid and salts and esters thereof (desirably lower alkyl (having 1 to 6 carbon atoms, for example) esters) being preferred. In the case of copolymers, the ratio of unit [I] to the other polymerizable monomer is not specifically limited, but a mol ratio of 1:0.3 or lower, particularly 1:0.1 or lower, is desirable.

The incorporation of a group that is photoreactive, and/or capable of bonding to the surface of the metal portion into the above polymer permits bonding of the base material and the polymer by means of the group present in the polymer. In the present invention, the term "photoreactive group" means a group that produces a radical when irradiated with light.

When a photoreactive group is incorporated into the polymer, the quantity incorporated of equal to or more than one group per molecule of polymer suffices, with equal to or more than two groups being desirable. However, when the quantity is excessively large, there is a risk of increased nonspecific adsorption. Thus, the quantity incorporated is desirably equal to or lower than 10 percent, preferably equal to or lower than 5 percent, of the number of carbon atoms (excluding carbon atoms in side chains) constituting the polymer. A desirable example of a photoreactive groups is an azide group (-N₃), but this is not a limitation. Specific examples of photoreactive groups that can be incorporated into the polymer are: phenyl azide groups, acetyl groups, and benzoyl groups. Phenyl azide groups are particularly preferred. The photoreactive group, such as an azide group, can be directly bonded to the polymer, or can be incorporated into the polymer by means of an optional spacer structure. Normal the latter is desirable due to of manufacturing. In the latter case, the spacer structure is not specifically limited. Examples are alkylene groups with 1 to 10 carbon atoms (which may be substituted with one or two hydroxyl groups) and phenylene groups (which may be substituted with one to three hydroxyl groups or alkyl groups having 1 to 4 carbon atoms).

The photoreactive group can be readily introduced into the polymer by the usual methods. For example, a polymer having a functional group and a diazide compound having a functional group reacting with the first functional group can be reacted to bond the azide group to the polymer. Polyethylene glycol is commercially available with amine groups or carboxyl groups on both ends. Thus, when employing polyethylene glycol, a desirable polymer, an azide group-containing polymer can be reacted with such commercially available functional group-containing polyethylene glycol, to incorporate an azide group. In the case of a polymer formed by polymerizing a monomer, such as a vinyl polymer, the vinyl monomer serving as the main structural unit of the vinyl polymer and a photoreactive vinyl monomer can be copolymerized to manufacture a photoreactive group-containing polymer. Specific examples of photoreactive group-containing vinyl polymers that can be obtained by this method are: poly((meth)acrylamide - photoreactive amide (meth)acrylate) copolymers, poly(glycidyl)(meth)acrylate - photoreactive (meth)acrylamidecopolymers, and (polyethylene glycol mono(meth)acrylate - photosensitive amide acrylate) copolymers. (Polyethylene glycol mono(meth)acrylate - photosensitive amide acrylate) copolymers are preferred.

Further, to incorporate a group that is capable of copolymerizing or coordinate bonding with the surface of the metal portion, the polymer desirably comprises a free carboxyl group. A free carboxyl group can be readily introduced, for example, by further copolymerizing a carboxyl group-containing vinyl monomer such as methacrylic acid or acrylic acid, The content of the free carboxyl group contained is desirably about 5 to about 50 mol percent based on the total number of mols of all units making up the polymer

Further examples of groups that are capable of coordinate bonding with the surface of the metal portion are carboxyl groups and amino groups. The free carboxyl groups contained in the above-described polymer can be utilized as is as carboxyl groups. Amino groups can be readily introduced by bonding the free carboxyl groups with a famine compound using a crosslinking agent such as carbodiimide.

The polymer layer can be formed by coating the polymer itself on the surface of the metal portion, or can be formed by coating a coating liquid containing the polymer on the surface of the metal portion, A photocrosslinking agent comprising at least two photoreactive groups per molecule can be contained in the coating liquid. In that case, the surface of the metal portion and the polymer can be bonded by the photocrosslinking agent, obviating the need to introduce a photoreactive group and/or a group that is capable of bonding with the surface of the metal portion into the polymer.

Within the photocrosslinking agent, the photoreactive groups produce radicals when irradiated with light, making it possible to form covalent bonds with the amino groups, carboxyl groups, and carbon atoms making up organic compounds. Thus, irradiating light after coating the coating liquid containing the photocrosslinking agent on the substrate makes it possible to bond the polymer, to the base material through the photocrosslinking agent, and makes it possible to form on the substrate a polymer layer having the effect of preventing nonspecific adsorption.

Examples of photoreactive groups contained in the above photocrosslinking agent are azide groups (-N₃), acetyl groups, benzoyl groups, and diazirine groups. Azide groups are particularly preferred because they release nitrogen molecule and produce nitrogen radicals when irradiated with light. The nitrogen radicals are capable of bonding not just with functional groups such as amino groups and carboxyl groups, but also with the carbon atoms constituting organic molecules, making it possible to form covalent bonds with most organic materials.

The photocrosslinking agent is desirably water soluble. The term "water soluble," as applied to the photocrosslinking agent, means the ability to produce an aqueous solution with a concentration of equal to or greater than 0.5 mM, desirably equal to or greater than 2 mM.

The photocrosslinking agent comprises at least two photoreactive groups per molecule. The number of photoreactive groups contained per molecule is, for example, 2 to 4, desirably 2 for 3. The photocrosslinking agent is desirably a diazide compound comprising two azide groups, preferably a water-soluble diazide compound. A desirable example of a photocrosslinking agent that can be employed in the present invention is the diazide compound denoted by general formula [II] below.

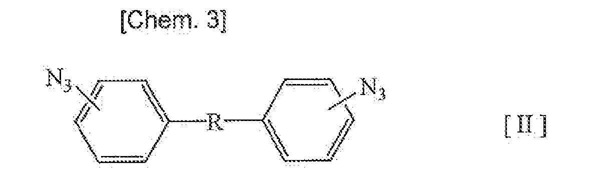

In general formula [II], R denotes a single bond or any group. -R-denotes a structure for linking two phenyl azide groups, and is thus not specifically limited beyond imparting the water solubility required for a diazide compound. Desirable examples of -R- are: a single bond (that is, the two phenyl azide groups are directly linked), an alkylene group having 1 to 6 carbon atoms (optionally containing one or two unsaturated bonds between carbons, it also being possible for one or two carbon atoms to be double bonded with oxygen to form carbonyl groups) (a methylene group being particularly preferred), -O-, -SO₂-, -S-S-, -S-, -R²...Y...R³- (where ... denotes a single bond or a double bond, Y denotes a cycloalkylene group with 3 to 8 carbon atoms, each of R² and R³ independently denotes an alkylene group with 1 to 6 carbon atoms (optionally containing one or two unsaturated bonds between carbons, it also being possible for the bond between the carbon atom on the end of the alkylene group and Y to be a double bond), it being possible for one or two of the carbon atoms to be double bonded with oxygen to form carbonyl groups). The cycloalkylene group may be substituted with one or more optional substituents (when substituted, one or two of the carbon atoms making up the cycloalkylene group are desirably double bonded with oxygen to form carbonyl groups, and/or are substituted with one or two alkyl groups having 1 to 6 carbon atoms). Each of the benzene rings in general formula [II] can be substituted with one or two substituents (such as halogens, alkoxyl groups having 1 to 4 carbon atoms, sulfonic acid, salts thereof, and other hydrophilic groups). Desirable specific examples of -R- are as follows: -, -CH₂-, -O-, -SO₂-, -S-S-, -S-, -CH=CH-, -CH=CH-CO-, -CH=CH-CO-CH=CH-, and CH=CH-,

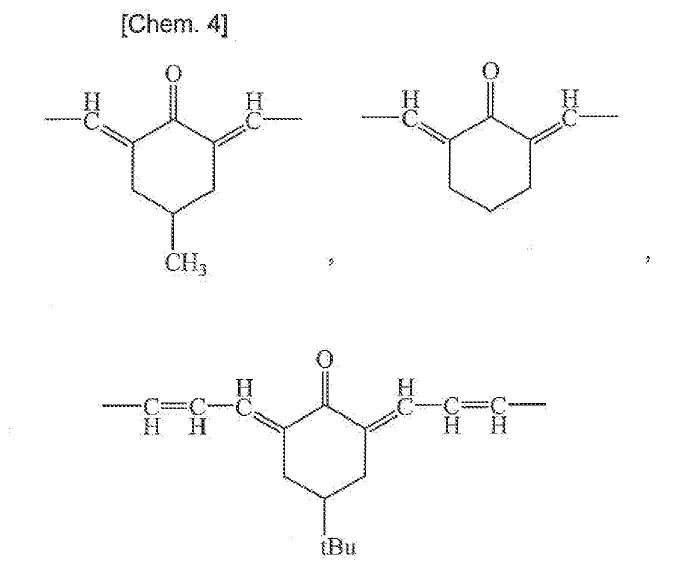

The following are desirable specific examples of diazide compounds,

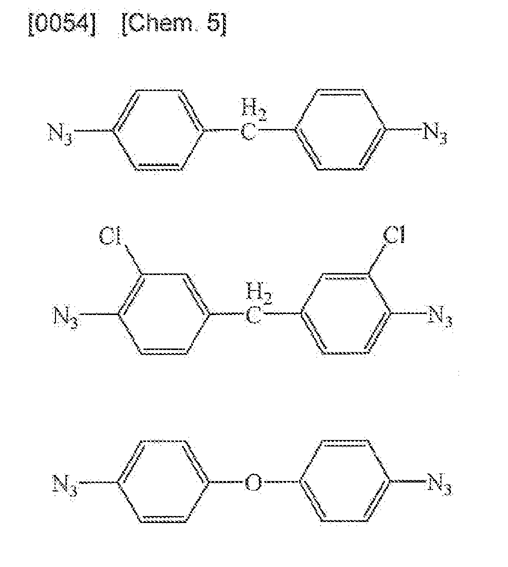

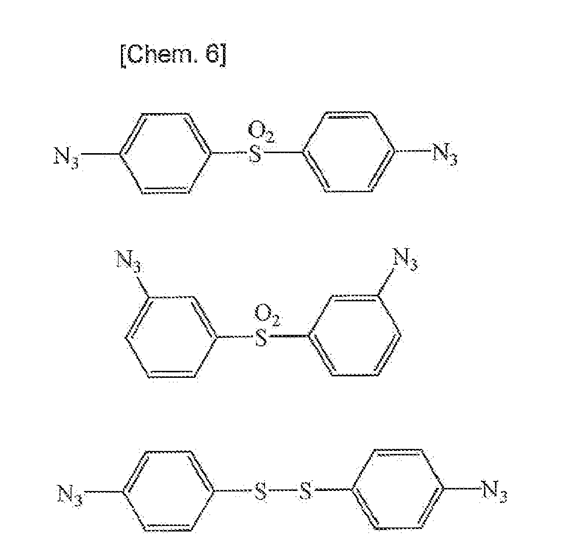

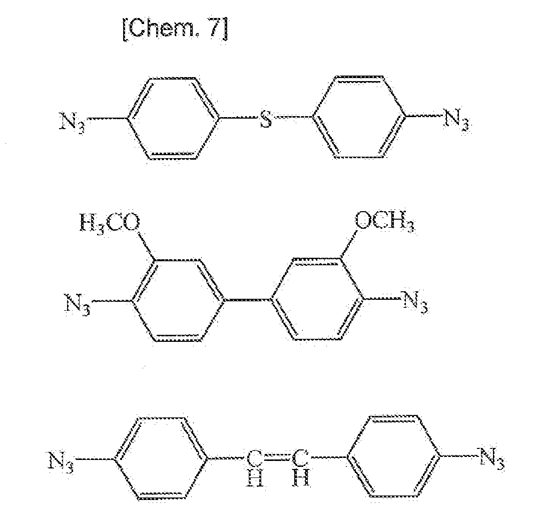

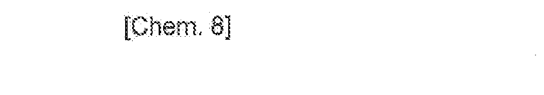

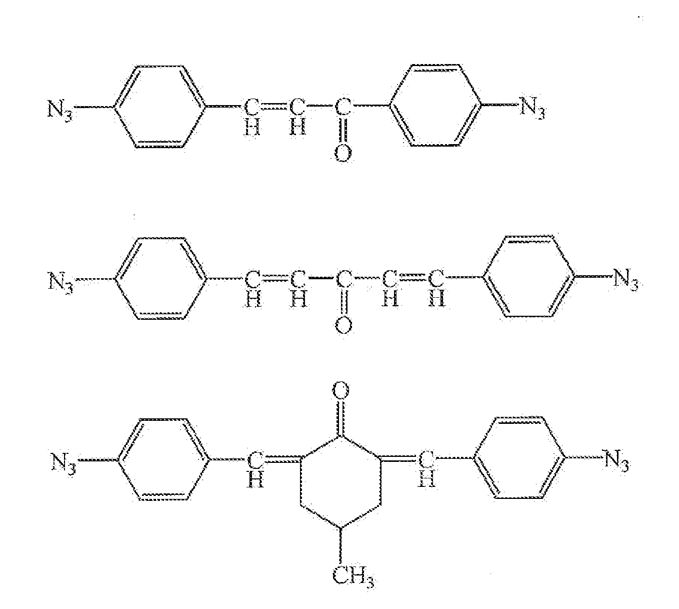

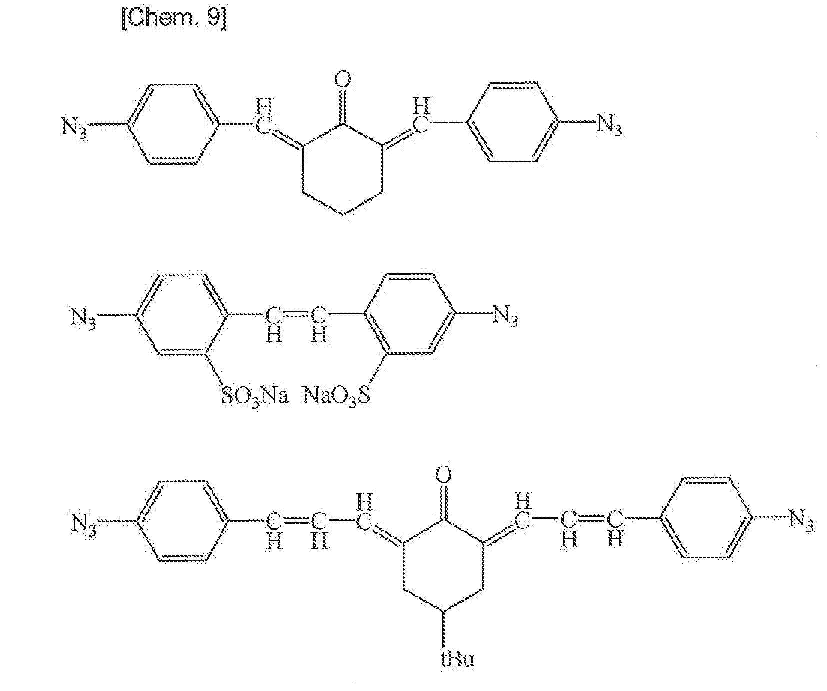

The polymer and photocrosslinking agent are known compounds per se and can be manufactured by known manufacturing methods. Some are also commercially available.

The polymer or the polymer and the photocrosslinking agent can, for example, be dissolved in a solvent to prepare a coating liquid. The solvent employed may be water, a lower alcohol (desirably ethanol) capable of being mixed with water in some proportion, or a mixture thereof. Of these, water is desirably employed as the solvent.

The concentration of the polymer and the photocrosslinking agent in the coating liquid are not specifically limited. The concentration of the polymer is, for example, 0.0001 to 10 mass percent, desirably 0.001 to 1 mass percent. The concentration of the photocrosslinking agent is, for example, 1 to 20 mass percent, desirably 2 to 10 mass percent, relative to the polymer. The method of coating the coating liquid on the surface of the metal portion is not specifically limited. For example, methods such as spin coating, spotting by micropipette or the like, pin-type spotting, and piezoelectric-type spotting can be employed. The film thickness is, for example, 0.01 to 10 micrometers, desirably 0.05 to 1 micrometer. The light transmittance of the polymer layer is desirably equal to or greater than 50 percent, preferably equal to or greater than 70 percent, and more preferably, 70 to 100 percent. Providing a polymer layer of high light transmittance on a metal portion with a high reflectance permits higher sensitivity detection. The light transmittance of the polymer layer can be measured, for example, by forming a polymer layer on a transparent glass substrate.

The substance that is immobilized on the substance-immobilizing substrate of the present invention is one that is to be detected by chemiluminescence. It is not specifically limited other than that it be detectable by chemiluminescence. Examples are polypeptides (including glycoproteins and lipoproteins), nucleic acids, lipids, cells (such as animal cells, plant cells, and microbe cells), and components thereof (including cellular organelles such as nuclei and mitochondria, and membranes such as cell membranes and unit membranes).

The present invention further relates to a substance-immobilized substrate wherein a substance is immobilized on the substance-immobilizing substrate of the present invention. The above substance is immobilized on the above metal layer on the substance-immobilized substrate of the present invention, which is used to directly or indirectly detect the immobilized substance by chemiluminescence.
The substance-immobilized substrate of the present invention will be described in further detail below.

The substance can be immobilized by coating a solution, obtained by adding the substance to be immobilized to a suitable solvent or buffer, on the metal portion of the substance-immobilizing substrate, directly or through the above polymer layer. In particular, a biopolymer having a specific structure, such as a protein, can be adsorbed onto chromium or molybdenum by causing it to undergo a partial conformational change.

In the present invention, a group capable of bonding to the metal portion or polymer layer can be incorporated into the substance to be immobilized. Further, immobilization can be achieved using a suitable intercalator. A mixture of the substance to be immobilized and a photocrosslinking agent having at least two photoreactive groups per molecule can be coated on the surface of the metal portion and irradiated with light to immobilize the substance. Of the above photoreactive groups, azide groups in particular release nitrogen molecules and produce nitrogen radicals when irradiated with light. The nitrogen radicals are capable of bonding not just with functional groups such as amino groups and carboxyl groups, but also the carbon atoms making up organic compounds, and are thus capable of immobilizing most organic materials.

When employing a photoreactive group, by coating the coating liquid and irradiating it with light, the photoreactive groups contained in the photocrosslinking agent can be converted to radicals, causing the metal portion or polymer layer and substance to be immobilized to bond with the photocrosslinking agent, thereby immobilizing the substance to be immobilized on the substrate. When bonding the substrate and polymer by means of a photoreactive group, the coating liquid for forming the polymer layer is coated on the metal portion and irradiated with light, desirably after having been dried. Next, a mixture of the substance to be immobilized and a crosslinking agent can be coated and irradiated with light. Alternatively, the mixture of the substance to be immobilized and the crosslinking agent can be coated and irradiated with light, without having irradiated light after coating the coating liquid for forming the polymer layer, to cause the photoreactive groups in the photocrosslinking agent and/or the polymer contained in the coating liquid to produce radicals. Details relating to the photocrosslinking agent employed in this regard are as set forth above.

The mixture of the substance to be immobilized and the photocrosslinking agent can be dissolved in a solvent and coated on the metal portion in a solution state, for example. The solvent employed may be water, a lower alcohol (desirably ethanol) capable of being mixed with water in some proportion, or a mixture thereof. Of these, water is desirably employed as the solvent.

The concentration of the substance to be immobilized in the sample solution is not specifically limited. It is, for example, 0.01 to 10 mass percent, desirably 0.05 to 1 mass percent.

When the sample solution contains a photocrosslinking agent, the concentration of the photocrosslinking agent is, for example, 0.1 to 20 mass percent, desirably 1 to 10 mass percent, of the substance to be immobilized.

The method of coating the sample solution is not specifically limited, The above-described coating methods and the like can be employed. When employing a photoreactive group, irradiation with light is conducted following coating, desirably after drying the solution that has been coated. The light can be any radiation that causes the photoreactive groups employed to produce radicals. In particular, when employing azide groups as photoreactive groups, ultraviolet radiation (with a wavelength of 300 to 400 nm, for example) is desirable. The period of irradiation is, for example, 1 to 15 minutes. The dose of radiation that is radiated is not specifically limited. The usual dose is about 1 mW to about 100 mW per cm². This irradiation with light causes the photoreactive groups contained in the photocrosslinking agent (or the photoreactive groups contained in the polymer when present therein) to produce radicals, causing the surface of the metal portion and the polymer layer, and the polymer layer and the substance to be immobilized, to bond through the photocrosslinking agent. In two-stage irradiation with light for causing the polymer layer and surface of the metal portion to bond, irradiation with light can be conducted under the similar conditions. Thus, a desired substance can be immobilized on the metal portion of the substrate, either directly or through the polymer layer.

When directly coating the sample solution on the metal portion, irradiation with light is not required, but can be conducted. Irradiation with light is thought to enhance the bonding strength of the substance to be immobilized and the surface of the metal portion by activating the surface of the metal portion.

In the present invention, microspotting can be employed to coat the above coating liquid and mixture (solution). Microspotting is a technique for applying a liquid on an extremely narrow region on a substrate. This method is commonly employed to fabricate DNA chips and the like, and the device for this method is commercially available. Thus, this technique can be readily conducted with the commercial available device. In the present invention, the coating liquid containing polymer or the polymer and, photocrosslinking agent can be coated over the entire surface of the substrate, and the mixture (solution) containing the photocrosslinking agent and the substance to be immobilized can be microspotted thereover, after which the entire surface of the substrate can be irradiated with light. Further, the above coating liquid can be microspotted and the mixture (solution) of the photocrosslinking agent and substance to be immobilized can be microspotted thereover, after which the entire surface of the substrate can be irradiated with light.

In the present invention, exposure to light can be selectively conducted through a photomask. When employing a photomask, photoreactive groups do not bond to the substrate or the substance to be immobilized in the portion that is not irradiated with light. Thus, washing can be conducted to remove the photocrosslinking agent and the substance to be immobilized. Accordingly, selective exposure to light through a photomask or the like permits the immobilization of a substance in any pattern. Since selective exposure to light permits the immobilization of a substance in any configuration such as microarrays, it is extremely useful.

In the present invention, following the immobilization of a desired substance as set forth above, it is desirable to wash the substrate by a known method to remove unreacted components and the like. A substrate on which a desired substance has been immobilized can then be obtained while inhibiting nonspecific adsorption.

The substance-immobilized substrate of the present invention is used to directly or indirectly detect the substance that has been immobilized by chemiluminescence. In detection by chemiluminescence, the substance that has been immobilized can be (directly) detected per se, or the substance that has been immobilized can be indirectly detected by detecting a substance specifically reacting with it. The term "chemiluminescence" refers to a highly sensitive, inexpensive detection method in which an enzyme-labeled antibody employed as a secondary antibody reacts with a substrate, and the energy that is thus generated is converted into luminescence and detected, Detection methods based on chemilummescence include the case where an antigen is immobilized on the surface of a base material and the quantity of antibody reacting specifically with it is measured, and the case where an antigen is immobilized on the surface of a base material, identical antigen and an antibody reacting specifically with that antigen are both added, and the quantity of the subsequently added antigen is measured (competitive method). The substance-immobilized substrate of the present invention can be employed in either of these methods,

The present invention further relates to an analysis method comprising directly or indirectly detecting by chemiluminesence a substance immobilized on a substrate. In the analysis method of the present invention, the substrate comprises a metal portion composed of at least one metal selected from the group consisting of chromium and molybdenum, or an alloy of such a metal, on at least a portion of the surface thereof, with the substance being immobilized on this metal portion. The details of the analysis method of the present invention are as set forth above. The analysis method of the present invention is suitable, for example, for use in analyzing antigen-antibody reactions.

### EXAMPLES

The present invention will be further described below through Examples. However, the present invention is not limited to the embodiments described in Examples.

### [Example 1]

### Preparation of chromium-coated glass substrate

A metal layer composed of chromium was formed by vacuum vapor deposition over the entire surface of one side of a glass base material. The glass base material was 0.7 mm in thickness and the chromium layer was about 1,100 Angstroms in thickness. In the above vapor deposition process, a chromium oxide layer about 500 Angstroms in thickness was formed between the chromium layer and the glass base material.

### [Example 2]

With the exception that the vapor deposition material was changed from chromium to molybdenum, a metal layer composed of molybdenum was formed by vacuum vapor deposition over the entire surface of one side of a glass base material by the same method as in Example 1.

### [Example 3]

### Preparation of BSA-immobilized chip (1)

### (1) Piranha processing

The chromium-coated glass substrate prepared in Example 1 was immersed for 5 minutes in piranha solution (sulfuric acid:30 mass percent hydrogen peroxide solution = 1:3 (mass ratio)), and then rinsed twice with MilliQ water.

### (2) Forming a polymer layer

Azide group-containing polyethylene glycol (also referred to as "Az-PEG", hereinafter) obtained by the method described in Example 3 of WO2004/004510 was dissolved in a 50 mass percent ethanol aqueous solution (quantity of Az-PEG: 0.15 mass percent) to prepare a coating liquid for forming a polymer layer. The coating liquid was applied dropwise to the surface of the chromium layer of the substrate, spin coated (4,000 rpm x 30 sec.), and dried under a reduced pressure of 0.09 MPa for 15 minutes. Subsequently, the surface of the polymer layer was irradiated with UV (7 minutes with black light (wavelength 300 to 400 nm)).

### (3) Immobilizing BSA

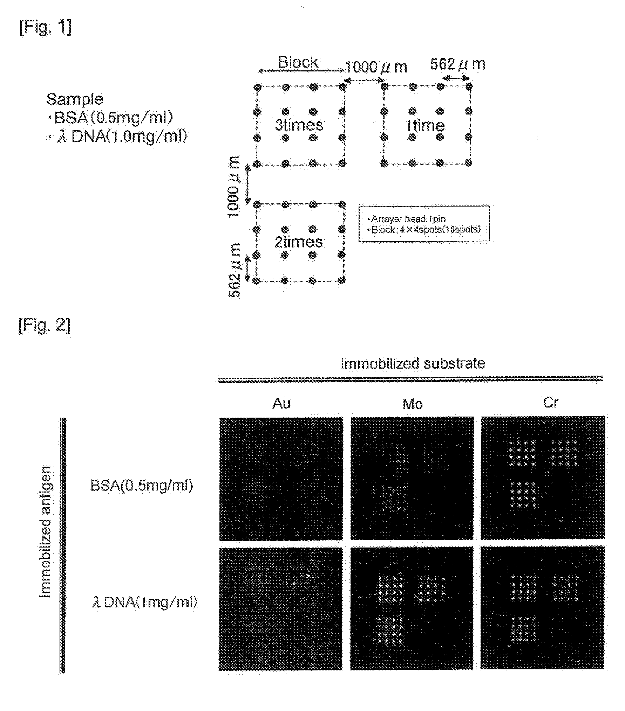

An antigen solution in the form of a BSA aqueous solution (BSA concentration: 0.5 mg/mL) was spotted on the polymer layer using the arrayer program shown in Fig. 1 with an arrayer. Subsequently, drying was conducted for 7 minutes at ordinary temperature and pressure. Next, the surface of the polymer layer containing the spots was irradiated with UV (7 minutes with black light (wavelength 300 to 400 nm)) to prepare a chip with immobilized antigen. The chip thus prepared was washed for 3 minutes in a mixer (strength 0) with cleaning solution composed of 0.1 mass percent Tween20 (registered trademark) added to phosphate buffer solution (also referred to as "PBS", hereinafter) and then dried for 5 minutes under a reduced pressure of 0.1 MPa.

### [Example 4]

### Preparation of a ADNA-immobilized chip (1)

With the exception that the antigen added to the antigen solution was changed to ADNA (ADNA concentration in antigen solution: 1 mg/mL), a ADNA-immobilized chip was prepared by the same method as in Example 3.

### [Example 5]

### Preparation of BSA-immobilized chip (2)

With the exception that the molybdenum-coated glass substrate prepared in Example 2 was employed, a chip with antigen immobilized on the molybdenum-coated glass substrate was prepared by the same method as in Example 3.

### [Example 6]

### Preparation of a ADNA-immobilized chip (2)

With the exception that the antigen added to the antigen solution was changed to ADNA (ADNA concentration in antigen solution: 1 mg/mL), a ADNA-immobilized chip was prepared by the same method as in Example 5.

### [Example 7]

### Analysis by chemiluminescence (1)

PBS-diluted primary reaction solutions (anti-BSA antibody solution: 100 ng/mL, anti-ADNA antibody positive serum: 100-fold dilution) were applied dropwise to the antigen-immobilized surfaces of the various chips prepared in Examples 3 to 6 and reacted for 20 minutes in a mixer (strength 0). Next, they were washed for 3 minutes in a mixer (strength 0) with PBS (containing 0.1 mass percent Tween20 (registered trademark)). Subsequently antibodies diluted with PBS containing 10 mass percent BSA (anti-rabbit IgG: 100-fold dilution, anti-human IgG: 4,000-fold dilution) were added dropwise and a reaction was conducted for 20 minutes in a mixer (strength 0). After washing for 3 minutes with PBS (containing 0.1 mass percent Tween20 (registered trademark)) in a mixer (strength 0), chemiluminescent reagent was added and the intensity of the chemiluminescence was measured.

### [Comparative Example 1]

With the exception that the substrate was replaced with a gold-coated glass substrate with one gold-coated surface, a BSA-immobilized chip was prepared by the same method as in Example 3 and subjected to chemiluminescence analysis by the same method as in Example 7.

### [Comparative Example 2]

With the exception that the substrate was replaced with a gold-coated glass substrate with one gold-coated surface, a λDNA-immobilized chip was prepared by the same method as in Example 4 and subjected to chemiluminescence analysis by the same method as in Example 7.

### Evaluation results

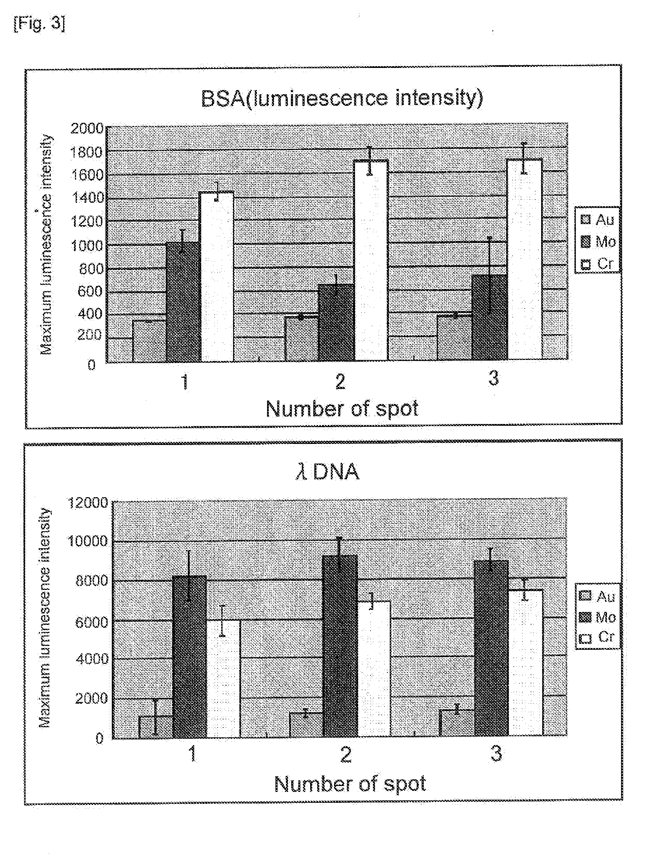

Fig. 2 shows CCD camera photographs (dark place, 50-fold magnification) of Example 7 and Comparative Examples 1 and 2. Fig. 3 shows graphs of the relation between the luminescence intensity and the number of spots obtained the first time, the second time, and the third time when the number of spots of antigen solution was changed in Example 7 and Comparative Examples 1 and 2.
As will be understood from the results of Figs. 2 and 3, the chromium-coated and molybdenum-coated substrates exhibited greater luminescence intensity and permitted more sensitive detection than the gold-coated substrate. As shown in Fig. 3, the gold-coated substrates, which exhibited no large increase in luminescence intensity even when the number of spots was increased, did not yield a luminescence intensity equivalent to that of the chromium-coated and molybdenum-coated substrates despite the increase in the number of spots.
When the BSA solution was spotted on the polymer layer, the polymer layer redissolved in the spot region. The BSA came into direct contract with the surface of the chromium layer and adsorbed physical which was thought to result in immobilization of the BSA on the chromium layer.

### [Example 8]

### Preparation of a BSA-immobilized chip (3)

With the exception that no piranha processing was conducted, a BSA-immobilized chip was prepared by the same method as in Example 3.

### [Example 9]

### Preparation of a BSA-inimobilized chip (4)

With the exceptions that the piranha processing conducted in Example 3 was replaced by an ozone treatment in which a chromium-coated substrate was irradiated with UV radiation for 10 minutes in an ozone atmosphere, a BSA-immobilized chip was prepared by the same method as in Example 3.

### (Example 10]

### Analysis by chemiluminescence (2)

The chips prepared in Examples 3, 8, and 9 were analyzed by chemiluminescence by the same method as in Example 7.

### Evaluation results

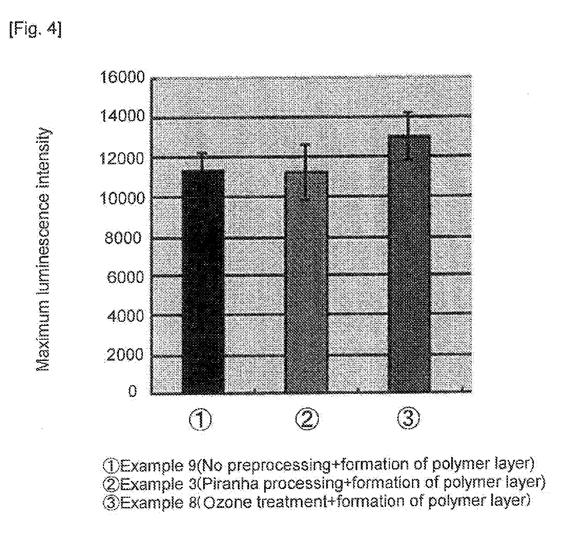

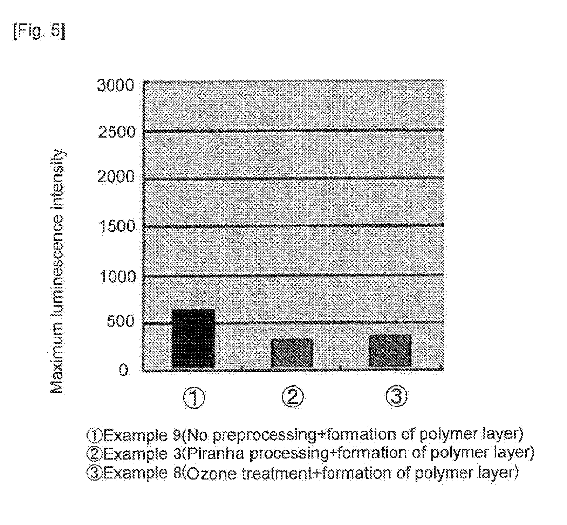

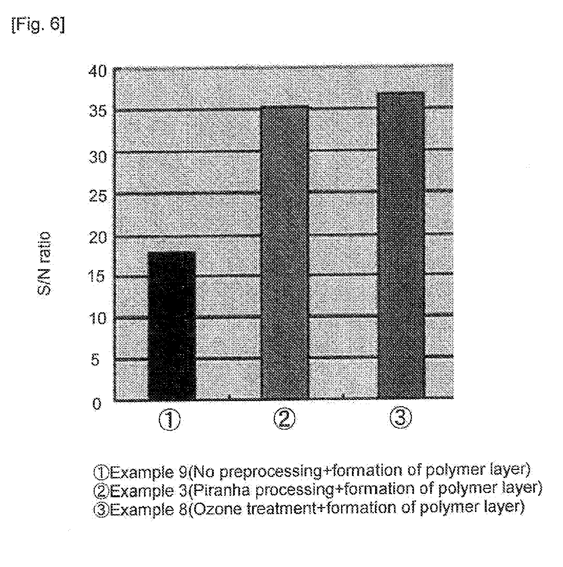

Fig. 4 shows the maximum spot luminescence intensity measured in Example 10 and Fig. 5 shows the maximum background luminescence intensity. Fig, 6 is a graph of the S/N ratio in Example 11 and Comparative Examples 3 and 4.
As shown in Fig, 4, highly intense luminescence was detected for the chips prepared in Examples 3, 8, and 9, As shown in Fig. 5, since the background luminescence intensity was low, nonspecific adsorption was found to have been inhibited. Since the intensity of the luminescence from spots was high and the intensity of the background luminescence was low in this manner in Example 11, highly sensitive analysis with a high S/N ratio was possible in the manner shown in Fig. 6.

### [Example 11]

### Antinuclear antibody test

An antinuclear antibody test was conducted for a cell solution by the method set forth below using a chromium-coated glass substrate prepared by the same method as in Example 1.

### (1) Forming of polymer layer

Az-PEG was dissolved in a 50 mass percent ethanol aqueous solution (quantity of Az-PEG: 0.15 mass percent) to prepare a coating liquid for forming a polymer layer, The coating liquid was applied dropwise to the surface of the chromium layer and spin coated (5,000 rpm x 30 sec.) and then dried under reduced pressure for 15 minutes at 0.09 MPa. Subsequently, the surface of the polymer layer was irradiated with UV (7 minutes with black light (wavelength 300 to 400 nm)).

### (2) Preparation of Ncu-immobilized chip

Nuclear fraction extracts (Nuc) of 125 mg/mL, 25 mg/mL, and 5 mg/mL were spotted on polymer layers with an arrayer, Subsequently, they were dried for 7 minutes at ordinary temperature and pressure and irradiated for 7 minutes with UV using the same black light as above to prepare chips. The chips prepared were washed for 3 minutes in a mixer (strength 0) with PBS (containing 0.1 mass percent Tween20 (registered trademark)) and then dried under reduced pressure for 5 minutes at 0.1 MPa.

### (3) Antinuclear antibody test

The level of protein in a nuclear fraction extract was quantified by the Bradford method. Protein solutions of known concentration were prepared based on the quantification results. A 10 percent quantity of bisazide was admixed to each of the protein concentrations. The protein solutions prepared were spotted with an arrayer on substrates. Following drying for 7 minutes at ordinary temperature and pressure, UV irradiation was conducted for 7 minutes using the above black light. Subsequently, the chips were washed for 3 minutes in a mixer (strength 0) with PBS (containing 0.1 mass percent Tween20 (registered trademark)) and then dried under reduced pressure for 5 minutes at 0.1 MPa. Following drying, serum diluted 100-fold was added dropwise to the chips and a reaction was conducted for 20 minutes in a mixer (strength 0). Subsequently, washing was conducted for 3 minutes in a mixer (strength 0) with PBS (containing 0.1 mass percent Tween20 (registered trademark)).
HRP-labeled anti-human IgG that was diluted 4,000-fold with PBS containing 10 mass percent BSA was applied dropwise to the washed chips and a reaction was conducted for 20 minutes in a mixer (strength 0). Subsequently, the chips were washed for 3 minutes in a mixer (strength 0) with PBS (containing 0,1 mass percent Tween20 (registered trademark)). Chemiluminescent, reagent was then added, photographs were taken by CCD camera (dark place, 50-fold magnification), and the intensity of the luminescence was measured,

### [Comparative Example 3]

The surface of the nitrocellulose layer of a nitrocellulose-coated glass substrate made by Whatman was irradiated with UV, washed, and subjected to blocking by incubation for 2 hours in blocking solution (4 mass percent skim milk). Subsequently, the nitrocellulose surface was antinuclear antibody tested by the same method as in Example 12.

### Evaluation results

Fig, 7 shows CCD camera photographs taken of Example 11 and Comparative Example 3, Fig. 8 shows the signal intensities measured for Example 12 and Comparative Example 3. Fig. 9 shows the signal intensity with the background subtracted out Fig. 10 shows a graph of the S/N ratio, In Figs, 7 to 10, "ANA+" means that antinuclear antibody was present and "ANA-" means that it was absent.
These results indicate that the chip of Example 12 exhibited a strong signal intensity and little background noise, indicating that highly sensitive analysis was possible at a high S/N ratio.
By contrast, Comparative Example 3, in which a nitrocellulose substrate was employed, the background noise was high and a good S/N ratio could not be achieved, even with blocking,

### INDUSTRIAL APPLICABILITY

The substance-immobilized substrate of the present invention can be suitably employed as a plate for immunological measurement on which an antibody, an antigen-binding fragment thereof, or an antigen is immobilized; as a nucleic acid chip, microarray, or the like comprising DNA or RNA immobilized on a substrate; or the like, but is not limited thereto. For example, it is suitable as a substrate on which entire cells or components thereof are immobilized,

### BRIEF DESCRIPTION OF THE DRAWINGS

[Fig. 1] A descriptive drawing of the program of the arrayer employed in Examples.
[Fig. 2] CCD camera photographs taken of Example 7 and Comparative Examples 1 and 2.
[Fig. 3] Graphs showing the relation between luminescence intensity and the number of spots of antigen solution in Example 7 and Comparative Examples 1 and 2.
[Fig. 4] A graph showing the maximum luminescence intensity from spots measured in Example 10.
[Fig. 5] A graph showing the maximum luminescence intense of the background measured in Example 10.
[Fig. 6] A graph showing the S/N ratio in Example 10.

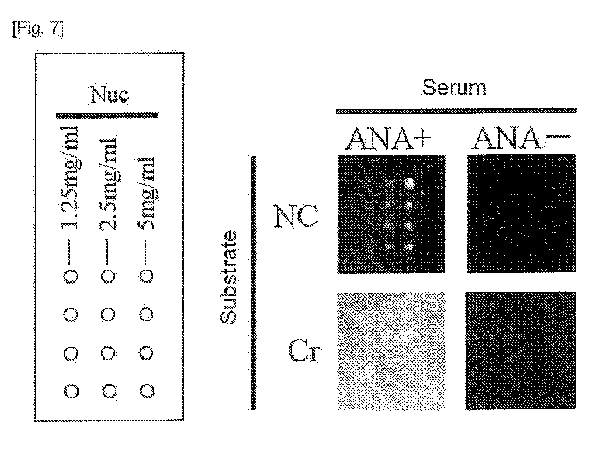

[Fig. 7] CCD camera photographs taken of Example 11 and Comparative Example 3.

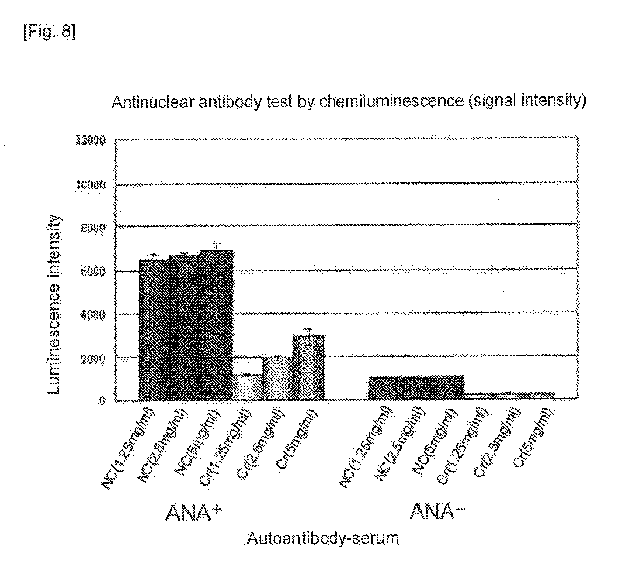

[Fig. 8] A graph showing the signal intensities measured in Example 11 and Comparative Examples 3.

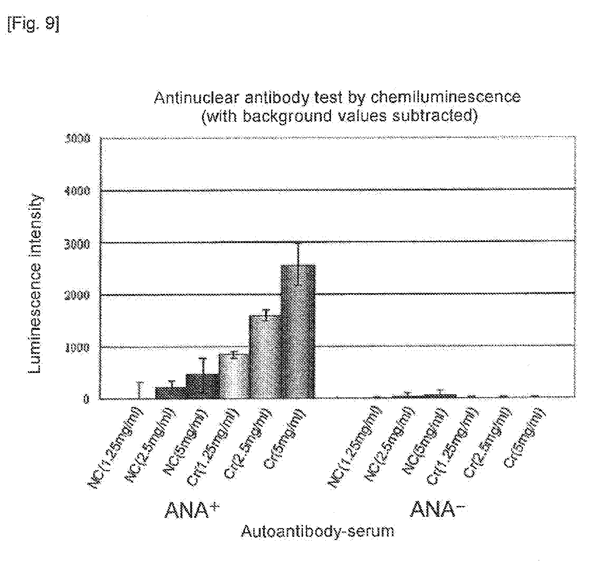

[Fig. 9] A graph showing the signal intensities (with background values subtracted) measured in Example 11 and Comparative Example 3.

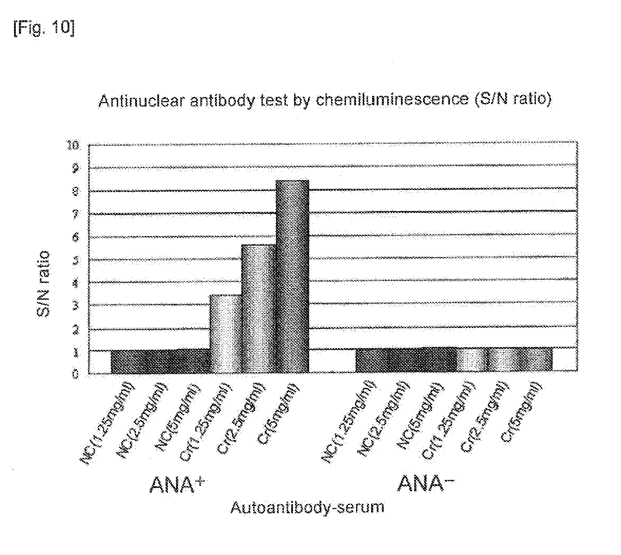

[Fig. 10] A graph showing the S/N ratio measured in Example 11 and Comparative Example 3.

## Claims

1. A substance-immobilizing substrate for immobilizing a substance to be directly or indirectly detected by chemiluminescence, comprising a metal portion composed of at least one metal selected from the group consisting of chromium and molybdenum, or an alloy of the metal on at least a portion of a surface of the substrate.

2. The substance-immobilizing substrate according to claim 1, comprising a layer of a polymer that is electrically neutral is a whole molecule, on at least a portion of a surface of the metal portion.

3. The substance-immobilizing substrate according to claim 1 or 2, wherein a surface of the metal portion has been subjected to piranha processing and/or ozone treatment.

4. The substance-immobilizing substrate according to any of claims 1 to 3, wherein the substance to be immobilized is at least one substance selected from the group consisting of polypeptides nucleic acids, lipids, cells, and components thereof.

5. A substance-immobilized substrate, **characterized in that**
a substance is immobilized on the substance-immobilizing substrate according to any of claims 1 to 3,
the substance is immobilized on the metal portion, and
the substrate is used for directly or indirectly detecting the immobilized substance by chemiluminescence.

6. The substance-immobilized substrate according to claim 5, wherein the substance is at least one substance selected from the group consisting of polypeptides, nucleic acids, lipids, cells, and components thereof.

7. An analysis method comprising directly or indirectly detecting by chemiluminescence a substance that is immobilized on a substrate,
where,in the substrate comprises a metal portion composed of at least one metal selected from the group consisting of chromium and molybdenum, or an alloy of the metal on at least a portion of a surface of the substrate, and
the substance is immobilized on the metal portion.

8. The analysis method according to claim 7, wherein the substrate comprises a layer of a polymer that is electrically neutral as a whole molecule on at least a portion of a surface of the metal portion.

9. The analysis method according to claim 7 or 8, wherein the surface of the metal portion is subjected to piranha processing and/or ozone treatment prior to immobilizing the substrate on the metal portion,

10. The analysis method according to any of claims 7 to 9, wherein the substance is at least one substance selected from the group consisting of polypeptides, nucleic acids, lipids, cells, and components thereof.

11. The analysis method according to any of claims 7 to 10, wherein the substance that is immobilized on the substrate is directly detected by chemiluminescence.

12. The analysis method according to any of claims 7 to 10, wherein the substance that is immobilized on the substrate is indirectly detected by detecting a substance reacting specifically with the substance that is immobilized on the substrate by chemiluminescence.
